Europäisches Patentamt

European Patent Office

Office européen des brevets

(11) Publication number: **0 074 842**
**B1**

# EUROPEAN PATENT SPECIFICATION

(45) Date of publication of patent specification: **08.05.85**

(51) Int. Cl.⁴: **A 61 M 3/00**

(21) Application number: **82304822.8**

(22) Date of filing: **13.09.82**

(54) A delivery device for delivering adjustable quantities of materials.

(30) Priority: **14.09.81 US 301671**

(43) Date of publication of application:
**23.03.83 Bulletin 83/12**

(45) Publication of the grant of the patent:
**08.05.85 Bulletin 85/19**

(84) Designated Contracting States:
**BE DE FR GB NL**

(56) References cited:
**WO-A-81/01104**
**GB-A-1 106 825**
**GB-A-1 550 308**
**US-A-4 026 287**

(73) Proprietor: **MERCK & CO. INC.**
**126, East Lincoln Avenue P.O. Box 2000**
**Rahway New Jersey 07065 (US)**

(72) Inventor: **Wood, William E.**
**35 Pope Street**
**Hudson Massachusetts (US)**
Inventor: **Rosen, Roy A.**
**RR No. 2**
**Woodstock Connecticut (US)**

(74) Representative: **Purvis, William Michael**
**Cameron et al**
**D. Young & Co. 10 Staple Inn**
**London WC1V 7RD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The invention relates to the delivery of adjustable quantities of material, and, more particularly, to the dispensing of measured amounts of material from a dispenser.

The dispensing of measured amounts of materials are required in many situations. One such situation is the dispensing of incremental dosages of gels or paste materials used as medicaments.

The typical dispensation of gel or paste medicaments has made use of devices which are difficult to adjust and, once adjusted, easily disturbed from their preset adjustment.

Such is the case, for example, with the commonly used, continuously threaded plunger. This device has a retaining ring that is threaded on the plunger and rotated to a desired position. The threading of the retaining ring is often a slow and cumbersome procedure. In addition, one positioned it is a relatively simple matter to disturb the present position. As a result, the accuracy and utility of this kind of device is far from satisfactory.

UK specification 1 550 308 discloses a syringe having a plunger with a stem portion of cruciform section and a setting ring surrounding the stem portion and having an arcuate tooth at its internal periphery, which tooth is engageable with any one of a plurality of cut out zones provided in one of the cruciform walls of the stem. By rotating the setting ring the tooth can be disengaged from a cut out zone thereby permitting the ring to be moved longitudinally of the stem and engaged with another of the cut out zones.

PCT specification WO81/01104 discloses a syringe with closely spaced indentations along the length of a plunger thereof and a dosage selection ring disposed around the plunger and having protuberances on its inside circumference which can be engaged in the indentations or disengaged therefrom by relative rotation of the dosage selection ring and the plunger.

According to the invention there is provided a delivery device comprising a housing having an outlet for material to be delivered therefrom; a plunger which can be reciprocated within the housing to effect delivery of the material; and means for controlling the extent of reciprocation of the plunger to regulate the amount of material expelled and delivered from the outlet, wherein the controlling means comprises a stop on the plunger on a portion thereof exterior to the housing and the stop is a collar surrounding the plunger and adjustably positioned on the plunger, the collar having at least one internal tooth engageable in transverse grooves in the plunger to secure the collar against longitudinal movement on the plunger characterised in that the collar is not rotatable with respect to the plunger and is formed of resilient material such that force applied to the collar can deform at least a portion of the collar to allow said at least one tooth on the collar to move out of engagement with the grooves in the plunger and enable the collar to be moved longitudinally of the plunger, the resilience of said portion of the collar causing said at least one tooth on the collar to be re-engaged with the grooves in the plunger when said force causing deformation is removed.

Preferably the plunger has an I-beam section with the grooves provided in one end flange thereof.

In one embodiment said portion of the collar comprises compressible side portions of the collar.

Internal prongs on the collar can define a track which embraces the opposite end flange of the I-beam section plunger to said one end flange thereof.

In another embodiment said portion of the collar comprises a flexible internal pawl which can bear against an opposite face of the plunger to the face in which the transverse grooves are provided, the flexible pawl being compressed by said force to elevate the tooth to a position above the level of the grooves.

Advantageously the grooves have first side walls which face in the direction in which the plunger is moved to cause delivery of the material with said first side walls lying in respective planes substantially perpendicular to the longitudinal axis of the plunger and second walls opposite to said first walls with said second walls forming inclined ramps and lying in respective planes angled at an angle in the range 15° to 45°, preferably 30°, to the longitudinal axis of the plunger.

The invention is diagrammatically illustrated by way of example in the accompanying drawings, in which:—

Figure 1 is a perspective view of a first embodiment of a delivery device in accordance to the invention;

Figure 2 is a cross-sectional view taken on line 2—2 of Figure 1;

Figure 3 is a cross-sectional view of a stop member on a plunger of the delivery device of Figure 2, taken on line 3—3 thereof;

Figure 4 is a cross-sectional view of the stop member of Figure 3, shown removed from its associated plunger;

Figure 5 is a partial cross-sectional view of a second embodiment of a delivery device according to the invention;

Figure 6 is a cross-sectional view taken on line 6—6 of Figure 5;

Figure 7 is a cross-sectional view corresponding to Figure 6 showing the side wall of a stop member being depressed in order to control the positioning of the stop member along an associated plunger;

Figure 8 is a partial sectional view showing the relation between an inlet of a housing of the delivery device of Figure 5 and the stop member on the plunger;

Figure 9 is a partial view showing the end of the plunger used in a delivery device according to the invention and its associated hub for a piston within the device; and

Figure 10 is a sectional view of an illustrative piston for engagement by the hub of Figure 9.

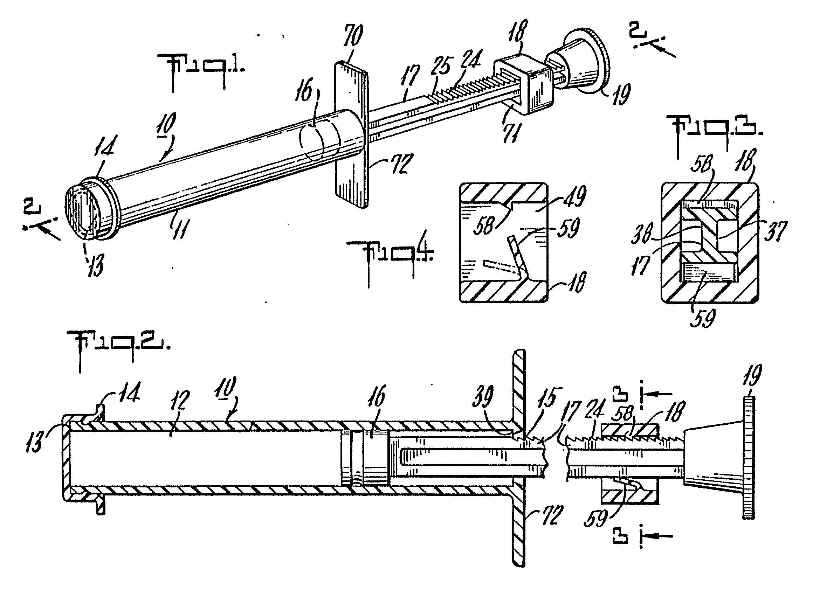

Referring to the drawings and firstly to Figures 1 to 4, a delivery device 10 includes a housing 11 and a reciprocating plunger 17. The housing 11 has an inlet 15 for receiving the plunger 17 at one end and at the opposite end includes an outlet opening 13 shown covered by a protective cap 14. Within the housing 11 is a piston 16 which is engagable and disengagable with respect to the plunger 17 in a manner specifically described below.

The plunger 17 additionally includes a stop member in the form of a collar 18 which is positionally adjustable along the plunger with respect to a set of grooves 24, 25. The exterior end of the plunger includes a boss or pommel 19 for the application of manual pressure to the plunger 17 in order to force it into the housing 11 and subsequently to withdraw it from the housing 11. The housing 11 illustratively includes an end flange 70 to facilitate gripping and positioning of the device 10 during use. In operation the plunger 17 is moved forward using the pommel 19 until a limiting face 71 of the stop member 18 engages a mating face 72 of the flange 70.

The housing 11 of Figure 1 is illustratively in the form of a cylindrical barrel, but any convenient cross section may be employed. The piston 16 is configured according to the cross section of the barrel and thus, for the cylindrical barrel of Figure 1, the piston 16 is shown with a circular cross section.

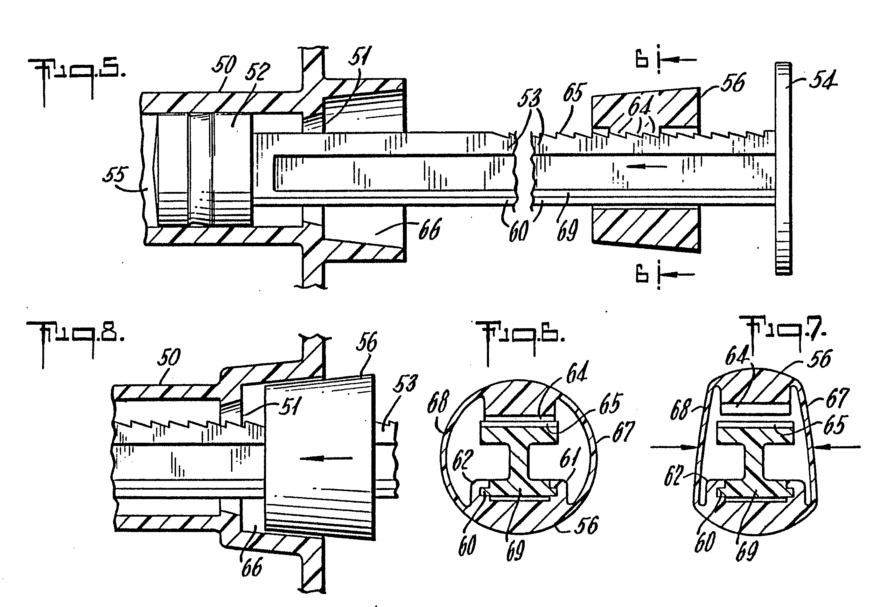

In the cross-sectional view of Figure 2, the piston 16 is shown in coupling engagement with the plunger 17. The manner in which the piston 16 can be disengaged from the plunger 17 is described below. In any event, when the plunger 17 is withdrawn towards the flange 70, after there has been engagement with the stop surface 72 of the flange by the collar 18, it ultimately engages a rim 39 which prevents the piston 16 from being withdrawn from the housing 11. In addition, the periphery of the piston 16 forms a seal with the rim 39 so that any material within the housing 11, for example a medicament 12, will not leak from the inlet 15, but can only be expelled from the outlet 13 after the protective cap 14 is removed. The seal provided by the piston 16, both when the piston is at an intermediate position within the housing 11 as illustrated in Figure 2 and when the piston is at the distal end of the housing 11, as the plunger is being withdrawn, not only prevents leakage but also avoids inadvertent contamination.

Control over the extent of expulsion of the medicament 12 by the reciprocating action of the plunger 17, when the cap 14 is removed, is also illustrated in Figure 2. For that purpose the plunger 17 is in the form of a rod with a set 24, 25 of depressions which advantageously are grooves extending transversely with respect to the longitudinal axis of the plunger 17. In particular the grooves 24, 25 are shown parallel to one another and form an angle in the range from another and form an angle in the range from about 30° to 90° with respect to the longitudinal axis of the rod 17. As illustrated in Figure 2 the angle of the grooves is 90° at the back of each groove, and the remainder of the groove is formed by an inclined ramp having an angle in the range between 15° and 45°, preferably 30°. The desired control is achieved by adjusting the collar 18 with respect to the grooves 24, 25.

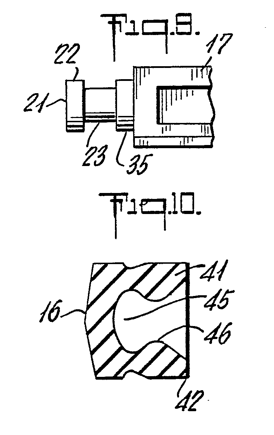

As seen in Figure 2, considered with Figures 3 and 4, the collar 18 includes a flexible internal pawl 59 which bears against a lower surface of the rod 17. The collar 18 also includes an upper internal projection 58 which engages one of the grooves 24, 25. Because of the flexible member 59 the projection 58 engages a selected one of the grooves 24, 25. When it is desired to adjust the collar, and thus the extent to which the plunger can be moved into the housing 11, the collar 18 is elevated, compressing the member 59, and elevating the projection 58 above the level of the grooves 24, 25. The collar can then be moved towards the stop surface 72 of the housing 11 or towards the pommel 19 at the distal end of the rod 17.

It is to be noted that the rod 17 includes indented sides 37 and 38 which can be marked with graduations that indicate the quantity of material delivered at the outlet 13 when the collar is at a particular setting and the rod has been pushed into the housing to the position where the stop face 71 of the collar 18 engages the stop face 72 of the flange 70. As ilustrated in Figure 4, the flexible member 59 is spring-like in its effect and, when the collar 18 is withdrawn from the rod 17, moves to a comparatively elevated position with respect to the normal operational position shown in broken lines in Figure 4. In will be appreciated that the surface of the rod 17 opposite the surface containing the grooves 24, 25 may also include depressions (not shown) for receiving the tip of the member 59 further to fix the position of the collar 18 at a specified location. The collar 18 includes an opening 49 which provides sufficient space for the projection 58 to clear the grooves 24, 25 when the collar is elevated and the flexible member 59 is compressed. The collar 18 is advantageously of a plastics material and the member 59 is accordingly a plastics, flexible pawl. The particular internal projection 58 is shown with a profile that meshes with the transverse grooves 24, 25, so that a ratcheting effect is provided and the collar 18 can be slid towards the stop surface 72, but is firmly seated against any rearward motion.

In using the delivery device 10, a suitable material such as a medicament 12, is loaded into the housing 11 after the cap 14 has been removed and the piston 16 has been positioned near the inlet 15. The rod 17 may be attached to the piston 16 either before or after the housing is loaded. The user then positions the collar 18 according to the amount of material that is to be ejected. As noted above, the recessed sides 37 and 38 may be marked to show the relationship between the positioning of the collar 18 on the rod 17 and the amount of material which will be expelled when the plunger is moved into the housing until the stop surfaces 71 and 72 are in contact with one another.

Once the stop collar is adjusted as desired, it is locked in position by the engagement of the projection 58 with a particular one of the grooves 24, 25. The cap 14 is then removed and the barrel of the housing 11 is applied as desired. For example, in the case of a medicament, the outlet 13 can be applied to a cavity of the body into which the contents are to be expelled. The user wraps his fingers about the portion of the housing 11 near the flange 70, desirably with his thumb and index finger against the flange 70, and forces the rod 17 into the housing 11 by applying pressure to the pommel 19 until the stop face 71 of the collar 18 engages the stop surface 72 of the flange 70.

It will be noted that the housing 11 can be loaded with a multiple dose of medicament, or a multiple charge of material. In that use the collar 18 is adjusted successively to permit the desired successive dose. Thus the collar is initially positioned away from the pommel 19 and then successively moved towards the pommel according to the successive doses that are required.

In the embodiment of Figure 5, a housing 50 has an inlet 51 which receives a plunger 53. A piston 52 is shown at the end of the plunger 53 in sealing engagement with the inner wall of the housing 50. The interior of the housing 50 is indicated generally by reference numeral 55. At the distal end of the plunger 53 there is included a pommel 54. It will be noted that the construction of Figure 5 generally resembles that of Figure 1 except that a stop collar 56 of different configuration is employed and that the end of the housing beyond the flange (corresponding to that of Figure 1) includes a concave seat 66 for mating with the collar 56 when the plunger 53 is fully operated.

The particular collar 56 of Figure 5 is shown in section in Figures 6 and 7, the latter showing the collar in the process of being adjusted. As seen in Figure 6, the collar 56 is an annular member that is mountedd circumferentially about the plunger 53. Sides 67 and 68 of the collar 56 are deformable, the rod 53 has an I-beam shape in cross section and the lower part of the collar has a track 60 degined by internal prongs 61 and 62 which embrace the lower beam portion 69 of the rod 53. The top portion of the collar has ratchet members 64 which can mesh with grooves 65 in the rod 53. When the sides 67 and 68 are pressed inwardly, the ratchet members 64 are lifted from the groove 65 so that the collar 56 can bd moved freely along the track 60.

When the rod 53 is moved into the housing the maximum penetration is determined by the collar 56 as shown in Figure 8.

The connection of the piston 16 to the plunger 17, and similarly of the piston 52 to the plunger 53, is illustrated in Figures 9 and 10. As shown specifically for the plunger 17 in Figure 9, the end of the plunger which is insertable into the housing 11 includes a hub 21 with annular ribs 22 and 35 which are spaced from one another by a shoulder 23. The hub 21 engages in the piston 16 which is illustrated in Figure 10. For that purpose, the piston 16 has a hollow central portion with convex walls 46 and exterior concave walls 42. The periphery of the piston 16 forms sealing lips 41. In operation the hub 21 is engaged in the piston 16 and seats in the opening 45 with the convex walls 46 in the interval between the annular ribs 22 and 35. When the plunger 17 is withdrawn from the housing 11, it is separable from the piston 16 by continued withdrawal of the plunger 17 after the piston 16 engages the rim 39.

**Claims**

1. A delivery device comprising a housing (11, 50) having an outlet for material to be delivered therefrom; a plunger (17, 53) which can be reciprocated within the housing to effect delivery of the material; and means for controlling the extent of reciprocation of the plunger to regulate the amount of material expelled and delivered from the outlet, wherein the controlling means comprises a stop on the plunger on a portion thereof exterior to the housing and the stop is a collar (18, 56) surrounding the plunger and adjustably positioned on the plunger, the collar having at least one internal tooth (58, 64) engageable in transverse grooves (24, 25, 65) in the plunger to secure the collar against longitudinal movement on the plunger characterised in that the collar (18, 56) is not rotatable with respect to the plunger (17, 53) and is formed of resilient material such that force applied to the collar can deform at least a portion (59, 67, 68) of the collar to allow said at least one tooth (58, 64) on the collar to move out of engagement with the grooves (24, 25, 65) in the plunger and enable the collar to be moved longitudinally of the plunger, the resilience of said portion of the collar causing said at least one tooth on the collar to be re-engaged with the grooves in the plunger when said force causing deformation is removed.

2. A delivery device according to claim 1, characterised in that the plunger (17, 53) has an I-beam section with the grooves (24, 25, 65) provided in one end flange thereof.

3. A delivery device according to claim 1 or claim 2, characterised in that said portion of the collar (56) comprises compressible side portions (67, 68) of the collar.

4. A delivery device according to claim 3 when appendant to claim 2, characterised by internal prongs (61, 62) on the collar (56) can define a track (60) which embraces the opposite end flange of the I-beam section plunger (53) to said one end flange thereof.

5. A delivery device according to claim 1, characterised in that said portion of the collar (18) comprises a flexible internal pawl (59) which can bear against an opposite face of the plunger (17) to the face in which the transverse grooves (24, 25) are provided, the flexible pawl being compressed by said force to elevate the tooth (58) to a position above the level of the grooves.

6. A delivery device according to any one of claims 1 to 5, characterised in that the grooves (24, 25, 65) have first side walls which face in the direction in which the plunger (17, 53) is moved to cause delivery of the material with said first side walls lying in respective planes substantially perpendicular to the longitudinal axis of the plunger and second walls opposite to said first walls with said second walls forming inclined ramps and lying in respective planes angled at an angle in the range 15° to 45°, preferably 30°, to the longitudinal axis of the plunger.

## Revendications

1. Un dispositif de distribution comprenant un carter (11, 50) pourvu d'une sortie pour la matière à distribuer; un plongeur (17, 53) qui peut être déplacé alternativement à l'intérieur du carter pour assurer la décharge de la matière; et un moyen pour commander la distance de déplacement alternative du plongeur afin de régler la quantité de matière expulsée et distribuée à la sortie, où le moyen de commande comprend un élément d'arrêt placé sur le plongeur sur une partie de celui-ci qui est extérieure au carter, l'élément d'arrêt étant une bague (18, 56) entourant le plongeur et étant positionnée de façon réglable sur le plongeur, la bague comportant au moins une dent intérieure (58, 64) pouvant s'angager dans des rainures transversales (24, 25, 65) du plongeur afin d'empêcher la bague de se déplacer longitudinalement sur le plongeur, caractérisé en ce que la bague (18, 56) ne peut pas tourner par rapport au plongeur (17, 53) et est formée d'une matière élastique de façon qu'une force appliquée à la bague puisse déformer au moins une partie (59, 67, 68) de la bague pour permettre au moins à ladite dent (58, 64) prévue sur la bague de se dégager des rainures (24, 25, 65) du plongeur et de permettre à la bague d'être déplacée longitudinalement sur le plongeur, l'élasticité de ladite partie de la bague faisant en sorte que ladite dent prévue au moins sur la bague soit réengagée dans les rainures du plongeur lors de la suppression de ladite force provoquant une déformation.

2. Un dispositif de distribution selon la revendication 1, caractérisé en ce que le plongeur (17, 53) a une section profilée en I, les rainures (24, 25, 65) étant prévues dans une aile extrême du profil.

3. Un dispositif de distribution selon la revendication 1 ou la revendication 2, caractérisé en ce que ladite partie de la bague (56) comprend des parties latérales compressibles (67, 68) de la bague.

4. Un dispositif de distribution selon la revendication 3, rattachée à la revendication 2, caractérisé par des branches internes (61, 62) de la bague (56) qui définissent une voie (60) qui enveloppe l'aile extrême du plongeur profilé en I (53) qui est opposée à l'aile extrême précitée.

5. Un dispositif de distribution selon la revendication 1, caractérisé en ce que ladite partie de la bague (18) comprend un cliquet interne flexible (59) qui peut s'appliquer contre une face du plongeur (17) qui est opposée à la face dans laquelle les rainures transversales (24, 25) sont prévues, le cliquet flexible étant comprimé par ladite force pour relever la dent (58) dans une position située au-dessus du niveau des rainures.

6. Un dispositif de distribution selon l'une quelconque des revendications 1 à 5, caractérisé en ce que les rainures (24, 25, 65) comportent des premières parois latérales qui sont orientées dans la direction de déplacement du plongeur (17, 53) pour assurer une décharge de la matière, lesdites premières parois latérales étant placées dans des plans respectifs sensiblement perpendiculaires à l'axe longitudinal du plongeur, et les secondes parois opposées auxdites premières parois, lesdites secondes parois formant des rampes inclinées et étant placées dans des plans respectifs faisant un angle compris entre 15 et 45°, de préférence de 30°, avec l'axe longitudinal du plongeur.

## Patentansprüche

1. Vorrichtung zum Abgeben mit einem Gehäuse (11, 50), das einen Auslaß für ein daraus abzugebendes Material hat, einem Kolben (17, 53), der im Gehäuse hin und her bewegbar ist, um eine Abgabe des Materials zu bewirken, und mit einer Einrichtung zur Regelung des Bereichs der Hin- und Herbewegung des Kolbens, um die ausgestoßene und aus dem Auslaß abgegebene Menge eines Materials einzustellen, wobei die Regeleinrichtung einen Anschlag auf dem Kolben an einem außerhalb des Gehäuses liegenden Teil aufweist, der Anschlag eine Hülse (18, 56) ist und die Hülse wenigstens einen Innenzahn (58, 64) hat, der in Eingriff mit Quernuten (24, 25, 65) im Kolben bringbar ist, um die Hülse gegen eine Längsbewegung auf dem Kolben festzulegen, dadurch gekennzeichnet, daß die Hülse (18, 56) bezüglich des Kolbens (17, 53) nicht drehbar ist, und aus einem nachgiebigen Material derart ausgebildet ist, daß die auf die Hülse einwirkende Kraft wenigstens einen Teil (59, 67, 68) der Hülse verformen kann, um zu ermöglichen, daß wenigstens ein Zahn (58, 64) auf der Hülse außer Eingriff von den Nuten (24, 25, 65) im Kolben kommt und die Hülse in Längsrichtung des Kolbens bewegbar ist, wobei das Federungsvermögen dieses Teils der Hülse bewirkt, daß der wenigstens eine Zahn auf der Hülse wiederum mit den Nuten in dem Kolben in Eingriff kommt, wenn die die Verformung bewirkende Kraft aufgehoben ist.

2. Vorrichtung zum Abgeben nach Anspruch 1, dadurch gekennzeichnet, daß der Kolben (17, 53) einen I-Profil-Querschnitt mit Nuten (24, 25, 65) hat, die an einem Endflansch desselben vorgesehen sind.

3. Vorrichtung zum Abgeben nach Anspruch 1 oder 2, dadurch gekennzeichnet, daß der Teil der Hülse (56) zusammendrückbare Seitenabschnitte (67, 68) der Hülse aufweist.

4. Vorrichtung zum Abgeben nach Anspruch 3 in Abhängigkeit von Anspruch 2, dadurch gekennzeichnet, daß Innenzacken (61, 62) auf der Hülse (56) eine Führungsbahn (60) bilden können, die den Endflansch des I-Profil-Kolbens (53) umgibt, der dem einen Endflansch gegenüberliegt.

5. Vorrichtung zum Abgeben nach Anspruch 1, dadurch gekennzeichnet, daß der Teil der Hülse (18) eine flexible innere Klaue (59) aufweist, die gegen eine Fläche des Kolbens (17) anliegt, die der Fläche gegenüberliegt, in der die Quernuten (24, 25) vorgesehen sind, und daß die flexible Klaue durch die Kraft zusammengedrückt wird, um den Zahn (58) in eine Lage oberhalb der Höhe der Nuten zu heben.

6. Vorrichtung zum Abgeben nach einem der Ansprüche 1 bis 5, dadurch gekennzeichnet, daß die Nuten (24, 25, 65) erste Seitenwandungen haben, die in die Richtung weisen, in der sich der Kolben (17, 53) bewegt, um ein Abgeben des Materials zu bewirken, wobei die esten Seitenwandungen jeweils in Ebenen im wesentlichen senkrecht zur Längsachse des Kolbens liegen, und daß zweite Wandungen den ersten Wandungen gegenüberliegen, wobei die zweiten wandungen schräge Flächen bilden und jeweils in Ebenen liegen, die unter einem Winkel im Bereich von 15° bis 45°, vorzugsweise von 30°, zur Längsachse des Kolbens angeordnet sind.

Fig.1.

70   17   25   24   18

16

14   10

13   11   72   71   19

Fig.3.

Fig.4.

49   58   18

58   59   38   37

18   17   59

Fig.2.

14   12   10   16   39   15   17   24   58   18   19

13   72   59

0 074 842

Fig.5.

Fig.6.

Fig.7.

Fig.8.

Fig.8.

22

21

23  35

17

Fig.10.

16

41
45
46

42